(19) Europäisches Patentamt

European Patent Office

Office européen des brevets

(11) Veröffentlichungsnummer: **0 518 908 B1**

(12) EUROPÄISCHE PATENTSCHRIFT

(45) Veröffentlichungstag der Patentschrift: **01.03.95**

(21) Anmeldenummer: **91905067.4**

(22) Anmeldetag: **06.03.91**

(86) Internationale Anmeldenummer:
**PCT/EP91/00419**

(87) Internationale Veröffentlichungsnummer:
**WO 91/13925 (19.09.91 91/22)**

Die Akte enthält technische Angaben, die nach
dem Eingang der Anmeldung eingereicht wurden
und die nicht in dieser Patentschrift enthalten sind.

(51) Int. Cl.⁶: **C08G  59/68**, C08G 59/70,
C09J 163/00

(54) **VERFAHREN ZUR POLYMERISATION VON EPOXIDVERBINDUNGEN.**

(30) Priorität: **09.03.90 DD 338525**
**09.03.90 DD 338526**

(43) Veröffentlichungstag der Anmeldung:
**23.12.92 Patentblatt  92/52**

(45) Bekanntmachung des Hinweises auf die
Patenterteilung:
**01.03.95 Patentblatt  95/09**

(84) Benannte Vertragsstaaten:
**AT BE CH DE DK ES FR GB GR IT LI LU NL SE**

(56) Entgegenhaltungen:
**CH-A- 629 230**      **DE-A- 2 334 467**
**US-A- 3 310 602**      **US-A- 3 367 913**
**US-A- 3 677 978**      **US-A- 4 473 674**
**US-A- 4 487 914**

(73) Patentinhaber: **RÜTGERSWERKE AKTIENGE-**
**SELLSCHAFT**
**Mainzer Landstrasse 217**

**D-60326 Frankfurt (DE)**

(72) Erfinder: **BÖTTCHER, Axel**
**Strasse der Kosmonauten 34**
**O-6900 Jena (DE)**
Erfinder: **UHLIG, Egon**
**Dietrichsweg 22**
**O-6900 Jena (DE)**
Erfinder: **FEDTKE, Manfred**
**Rosenweg 14**
**D-4200 Merseburg (DE)**
Erfinder: **DÖRING, Manfred**
**Wiesenstrasse 10**
**O-6900 Jena (DE)**
Erfinder: **DATHE, Klaus**
**Eibenweg 9**
**O-6900 Jena (DE)**
Erfinder: **NESTLER, Bernd**
**Leipziger Strasse 10**
**O-6900 Jena (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Polymerisationsverfahren von epoxidischen Verbindungen mittels einer die Reaktion beschleunigenden Lewis-Base, welches durch die Art seines ablaufenden Reaktionsmechanismus es ermöglicht, die Polymerisation der epoxidischen Verbindungen so zu gestalten, daß universell einsetzbare Klebe-, Schicht- und Formmassen für die Medizin, den Umweltschutz, besonders aber für die Justierung von Klebeteilen in der optischen Industrie hergestellt werden können.

Zudem wird die lösungsmittelfreie Herstellung von Epoxidharzmassen realisiert. Diese Epoxidharzmassen sind u. a. als Form- und Schichtmaterial anwendbar und universell, besonders aber in der Medizin, im Umweltschutz und in der optischen Industrie einsetzbar. Insbesondere aber eignet sich das Polymerisationsverfahren bei der Herstellung von Prepregs und Laminaten mit Epoxidharz als Bindemittel, insbesondere zur Herstellung von Basismaterialien für hochwertige elektrische Leiterplatten und für Hochleistungsverbundwerkstoffe.

Es ist bekannt, daß Lewis-Basen, wie beispielsweise Imidazole sehr reaktive Beschleuniger von Polymerisationsreaktionen Epoxid- bzw. Oxiran-gruppen enthaltender Materialien sind (Ricciardi, F. et. al., J. Polym. Sci. Polym. Chem. Ed 1983, 21, 1475-90, Farkas, A. et. al., J. Appl. Polym. Sci. 1968, 12, 159-68). Als nachteilig an diesen Lösungen erwies sich besonders die starke Temperaturerhöhung während des Polymerisationsvorganges, die zu einer unerwünschten Verfärbung der Polymermassen und zu Inhomogenitäten innerhalb der Polymermassen führt.

Solch eine Temperaturerhöhung tritt auch auf, wenn aus Imidazol oder aus substituierten Imidazolen und der Epoxidverbindung, wie in US 4 487 914 beschrieben, ein Addukt hergestellt wird, das gemeinsam mit einem Metallsalz zu einem vorkondensierten harzartigen Produkt umgesetzt und als Härter verwendet wird.

In der DE-OS 28 10 428 wird eine Möglichkeit aufgezeigt, die große Reaktivität der eingesetzten Lewis-Basen gegenüber polymerisationsfähigen epoxidischen Verbindungen durch Zugabe von Lösungsmitteln "aus der Gruppe Methanol, Ethanol oder Gemischen davon" zu mildern.

Die Problematik dieser Lösung besteht in dem Erfordernis zusätzlicher Verarbeitungsschritte zur Entfernung des Lösungsmittels nach der Polymerisation sowie im Entstehen eigenschaftsmindernder Hohlräume im Polymer, die die Wasseraufnahmefähigkeit des Polymers steigern.

In den Patentschriften US 3,638,007; US 3,792,016; US 4,101,514 und DE 23 00 489 wurden Imidazol-Metall-Verbindungen in ihrer Wirkung als Lewis-Base zur reaktiven Beschleunigung von Polymerisationsreaktionen untersucht. Diese Verbindungen stellen sehr stabile Koordinationspolymere dar und spalten aus diesen Gründen auch erst bei relativ hohen Temperaturen Imidazol ab, das dann seine Wirkung als Lewis-Base entfaltet. Es wird unterhalb 170 °C keine beschleunigende Wirkung auf die Epoxidmonomere beobachtet.

Die Problematik bei Polymerisationsreaktionen mit Epoxiden bei derartig hohen Temperaturen besteht im Auftreten von Ringspaltungsprodukten, die einen schlechten Netzwerkaufbau bewirken und somit negative Polymereigenschaften, wie Schwindungsverhalten, Wasseraufnahme u. ä. bedingen.

Durch den Einsatz von Hilfsbasen können die Temperaturen gemildert und die Beschleunigungswirkung somit gesteuert werden.

Der Einsatz derartiger Hilfsbasen besitzt jedoch folgende Nachteile:
- hohe ökonomische Aufwendungen, da diese Hilfsbasen sehr kostenintensiv sind
- die Möglichkeit des Reagierens der Hilfsbasen selbst mit den Epoxidsystemen.

In der Patentschrift US 3,677,978 wird die Verwendung von Imidazol-Komplexen verschiedenster Metallsalze, z. B. $CuCl_2$, $CuSO_4$, $NiCl_2$ und $CoCl_2$, die 1 bis 6 Mol Imidazol pro Mol Metallsalz enthalten, als latente Beschleuniger beschrieben. Nachteilig dabei ist,
- die hohe Geltemperatur von ca. 160 bis 260 °C, die zu Inhomogenitäten und Schwarzfärbung führt,
- die nichthomogene Löslichkeit des zu polymerisierenden Reaktionsgemisches.

Auch in der Patentschrift US 3,553,166 wird die Verwendung von Imidazol-Komplexen sowie einer zusätzlichen stickstoffhaltigen Verbindung zur Härtung von Epoxidharzen dargestellt. Die erforderliche Verarbeitungstemperatur liegt gleichfalls um 180 °C.

Bekannt ist auch die Überführung von Imidazolen in latente Härter und Beschleuniger durch Salzbildung mit unterschiedlichen Säuren, wie Polycarbonsäure (US 3,746,686), Isocyanursäure (DE 28 11 764), Phosphorsäure (US 3,632,427, US 3,642,698, US 3,635,894). Die besondere Problematik dieser Lösungen besteht:
- in der hohen toxischen Wirkung der Imidazol-Verbindungen
- im vorzeitigen Polymerisationsabbruch durch die Anwesenheit von Anionen

- damit verbunden im Auftreten eines erhöhten Monomeranteils im polymeren Epoxid und solcher Parameter, wie Ausschwitzung und erhöhte Wasseraufnahme.

Aus DE 20 19 816 und US 4,137,275 ist der Einsatz von Acetylacetonatokomplexen zur Polymerisation von Epoxidverbindungen auch in Gegenwart von Carbonsäureanhydriden bekannt. Nachteilig an diesen Lösungen ist ebenfalls die hohe Verarbeitungstemperatur größer 150 °C. Neben der bereits aufgeführten Problematik bei Polymerisationsreaktionen mit Epoxiden bei derartig hohen Temperaturen bedeutet dies, daß es zu enormen Aufwendungen in der Verarbeitung führt und die Anwendungsbreite stark einschränkt.

In der DE 25 25 248 wird beispielsweise Cr (acac)$_3$; (acac = acetylacetonat) als Härtersystem für Epoxidharzmassen beschrieben. Nachteilig dabei ist, daß neben der Oxiransauerstoffverbindung zusätzlich eine labile Wasserstoffverbindung vorhanden sein muß, um eine Polymerisation oder Aushärtung zu erreichen. Ein weiterer Nachteil derartiger Mischungen besteht darin, daß oftmals die Polymerisation direkt nach der Härterzugabe einsetzt. Es können somit keine lagerfähigen Polymermischungen gewährleistet werden.

Auch in US 4 473 674 wird ein Co(III)-acetylacetonat als Härterbestandteil für Epoxidharze verwendet. Da das Acetylacetonat in Gegenwart eines Lösungsmittels sowie eines aromatischen Diamins und eines Phenolnovolaks verwendet wird, muß die Aushärtung in aufwendiger Weise so geführt werden, daß das Lösungsmittel freigesetzt werden kann und erst anschließend bei einer entsprechend höheren Temperatur die Aushärtung erfolgt. Und zwar wird im ersten Schritt bei erhöhter Temperatur unter Vakuum in einer Zeit von 45 bis 55 Minuten entgast. Anschließend wird die Temperatur unter Normaldruck innerhalb von mehreren Stunden auf die Härtungstemperatur von 150 °C aufgeheizt und danach noch zwei bis drei Stunden bei 175 bis 177 °C nachgehärtet ohne die Produktqualität zu beeinträchtigen.

Die durch DE-A 2 334 467 beschriebenen Acetylacetonato-Härtungskatalysatoren für Epoxidharze sind, wie im Beispiel 1 der Schrift zu sehen ist, extrem aufwendig und daher unwirtschaftlich herzustellen. Außerdem ist die Umsetzung der Einzelkomponenten miteinander nicht exakt stöchiometrisch, so daß der entstehende Härter in seiner Qualität schwankt. Daher eignen sich diese Härter lediglich für Druckfarben und Beschichtungen, jedoch nicht zur Herstellung hochwertiger Produkte.

Es wurde auch versucht, die Reaktivität von primären Aminen, die als Härter für Epoxidharze seit langem bekannt sind, durch Komplexbildung zu senken. Entsprechende Diaminkomplexe, die aus CH-A 629 230 bekannt sind, weisen jedoch, wie aus Tabelle I zu ersehen ist, sehr hohe Anspringtemperaturen (120 °C) und sehr lange Nachhärtungszeiten (3 h) auf, die auch noch bei relativ hohen Temperaturen erfolgen.

Bei den aus US-A 3 310 602 bekannten Komplexen aromatischer Amine handelt es sich um latente Härter. Zur schnellen, wirtschaftlichen Aushärtung der Harze muß die Temperatur, wie aus den Beispielen zu ersehen ist, jedoch auf 150 °C erhöht werden, was zu den gleichen, oben beschriebenen Nachteilen führt. Entsprechendes gilt für die aus US-A 3 367 913 bekannten Aminophenol-Komplexe, die erst bei etwa 150 °C (300 °F) aushärten.

Die generelle Problematik bei der Verwendung von Imidazolen als Beschleuniger besteht u. a. darin, daß bei ihrem Einsatz unabhängig, ob in freier oder gebundener Form, sofort eine vollständige Durchhärtung erfolgt. Daher existieren immer zwei Zustände der Harzmasse, zum einen der unvernetzte und zum anderen der völlig vernetzte Zustand. Dies gewährleistet zwar einen Einsatz als Form- und Schichtmaterial mit optimalen Gelzeiten, die auch je nach Art des verwendeten Imidazols variiert werden können, jedoch ist es nicht möglich nach Zugabe des Beschleunigers eine Reaktionsphase der langsamen Aushärtung und damit verbunden eine individuelle Verarbeitungsphase, beispielsweise eine Justierphase beim Einpassen von zu verklebenden Teilen, vor der eigentlichen Härtungsphase einzusteuern.

Ziel der Erfindung ist ein Verfahren zur Polymerisation von Epoxidverbindungen mittels einer Lewis-Base, welches die Herstellung von kostengünstigen, umweltfreundlichen und nichttoxischen Epoxidharzmassen mit optimalen Gelzeiten auf Grundlage von Metallkomplexen ermöglicht.

Der Erfindung liegt die Aufgabe zugrunde, ein Verfahren zur Polymerisation von Epoxidverbindungen mittels einer Lewis-Base als Initiator zu schaffen, welches die latente Initiierung der Polymerisation durch thermische Abspaltung des Initiators aus dem Komplex bei Temperaturen kleiner 140 °C insbesondere ab 50 °C realisiert.

Die Lösung der Aufgabe erfolgt durch ein Verfahren zur Polymerisation von Epoxidverbindungen gemäß der Ansprüche 1 bis 5, durch Mischungen von Epoxidverbindungen, die erfindungsgemäß polymerisiert werden, gemäß der Ansprüche 6 und 7, sowie durch die bevorzugten Verwendungen der erfindungsgemäßen Mittel gemäß der Ansprüche 8 bis 12.

Erfindungsgemäß wird die Aufgabe durch ein Verfahren zur Polymerisation von Epoxidverbindungen mittels einer die Reaktion beschleunigenden Lewis-Base dadurch gelöst, daß 100 Gewichtsteile der Epoxidverbindung mit 0,01 bis 50 Gewichtsteilen, bevorzugt mit 1 bis 10 Gewichtsteilen einer Metallkomplexverbindung der allgemeinen Formeln

$ML_xB_y$ , oder M $[SR]_x$ $B_z$

versetzt wird und Energie zugeführt, wobei

- M ein Metallion der II. und III. Hauptgruppe sowie der Nebengruppen des Periodensystems,
- L ein chelatbildender Ligand aus der Gruppe der Dioxime, alpha- und $\beta$-Hydroxycarbonylverbindungen oder enolisierbaren 1,3 Diketone,
- SR ein Säurerest einer anorganischen Säure
- B eine Lewis-Base
- x 1 oder 2
- y eine natürliche Zahl im Bereich von 1 bis 5
- z eine natürliche Zahl im Bereich von 7 bis 8

sind, mit der Maßgabe, daß die Lewis-Base keine mehrwertige phenolische Verbindung ist.

Als Epoxidverbindungen können im erfindungsgemäßen Verfahren alle Epoxidverbindungen mit mehr als einer Epoxidbindung eingesetzt werden.

Bevorzugte Epoxidverbindungen sind Polyphenol-Glycidylether, z. B. epoxidierte Novolake oder die Reaktionsprodukte aus Epichlorhydrin und Bisphenol A oder Bisphenol F. Derartige Epoxidharze haben ein Epoxid-Äquivalent von 160 bis 500. Die vorstehenden polyfunktionellen Epoxidverbindungen (dieser Ausdruck schließt auch den Begriff Epoxidharz ein) können einzeln oder im Gemisch, gegebenenfalls in Gegenwart von Lösungsmitteln nach dem vorliegenden Verfahren polymerisiert werden. Sie können auch im Gemisch mit Monoepoxyden (sogenannten Reaktivverdünnern) eingesetzt werden.

Als Metalle (M) können im Prinzip alle Metallionen dienen, insbesondere die Ionen der 2. und 3. Hauptgruppe des Periodensystems der Elemente sowie die Metallionen der Nebengruppenelemente.

Bevorzugte Metallionen sind Cobalt-, Nickel-, Eisen-, Zink- oder Mangan-ionen.

Liganden (L) sind entweder chelatbildende Liganden Chelatbildende Liganden sind organische Verbindungen, die mindestens zwei Atomgruppierungen enthalten, die als Elektronendonatoren wirken. Beispiele hierfür sind Dioxime, alpha- und $\beta$-Hydroxycarbonylverbindungen oder auch enolisierbare 1,3-Diketone.

Bevorzugte Chelatliganden sind Acetylaceton, Benzoylaceton oder Dipivaloylmethan.

Als Säurerestion (SR) kann ein beliebiger Säurerest einer anorganischen Säure dienen.

Als Lewis-Basen (B) für die erfindungsgemäß einzusetzenden Metallkomplexverbindungen eignen sich alle nucleophilen Moleküle oder Ionen, die ein einsames Elektronenpaar aufweisen. Beispiele sind Pyridin- oder Imidazolverbindungen, Ether, einschließlich cyclischen Ethern wie z. B. Tetrahydrofuran, Alkohole, Ketone, Thioether oder Merkaptane, ausgenommen phenolische Verbindungen.

Lewis-Basen können in den Komplexen gemäß der Formel $ML_xB_y$ aber auch CH-acide Verbindungen sein, die als Lewis-Basen vorliegen, d. h., CH-acide Verbindungen, bei denen ein Proton abgespalten ist. Beispiele für derartige CH-acide Lewis-Basen sind CH-acide Pyridine, Malonsäure-diester oder -dinitril, Acetessigsäureester, Cyanessigsäureester oder Nitromethan.

Der Ladungsausgleich der Metallkationen der einzusetzenden Metallkomplexverbindungen kann sowohl durch die Liganden, als auch durch ionische Lewis-Basen erfolgen. Dabei versteht es sich, daß sich die Zahl der ladungstragenden Liganden reduziert, wenn der Komplex ionische Lewis-Basen enthält.

Eine weitere Ausgestaltung der erfindungsgemäß eingesetzten Komplexe besteht darin, daß die CH-aciden Lewis-Basen über Stickstoff- und/oder Sauerstoff- und/oder Schwefel- und/oder Phosphor-Atome bzw. Wasserstoffbrücken an die Metallchelat-Verbindung gebunden sind.

Diese Metallkomplexverbindungen werden in an sich bekannter Weise durch Umsetzung der entsprechenden Metallsalze mit den gewünschten Liganden und Lewis-Basen erhalten.

Besonders geeignete Metallkomplexverbindungen sind folgende Metallkomplexe:

Bis(acetylacetonato)-Cobalt-II-diimidazol,
Bis(acetylacetonato)-Nickel-II-diimidazol,
Bis(acetylacetonato)-Zink-II-diimidazol,
Bis(acetylacetonato)-Mangan-II-diimidazol,
Bis(acetylacetonato)-Eisen-II-diimidazol,
Bis(acetylacetonato)-Cobalt-II-didimethylimidazol,
Bis(acetylacetonato)-Cobalt-II-dibenzimidazol,
Bis(acetato)-Cobalt-II-diimidazol,
Bis[2-ethylhexanato] -Cobalt-II-diimidazol und
Bis(salicylaldehydo)-Cobalt-II-diimidazol.

Erfindungsgemäß werden die Komplexe mit den Epoxidverbindungen bei einer Temperatur unterhalb der Initiierungstemperatur, d. h. bevorzugt im Bereich von Raumtemperatur bis 50 °C gemischt.

4

Die Mischungen sind in diesem Bereich lagerfähig und können zu Form- oder Gießmassen, Klebstoffmischungen oder Prepregs verarbeitet werden.

Die Härtung der Epoxidverbindung erfolgt dann durch Energiezufuhr, wobei die Temperatur über die Initiierungstemperatur der Komplexe steigt.

Die Zuführung von Energie kann in Form von thermischer Energie, Licht, Mikrowellen, Strahlung, Laserenergie etc. erfolgen.

Die Vorteile der Erfindung ergeben sich im wesentlichen dadurch, daß

- eine Möglichkeit gegeben wird, den Metallkomplex in der polymerisierbaren epoxidischen Verbindung, bzw. in dem polymerisierbaren Epoxidgemisch unterhalb der Polymerisationsinitiierungstemperatur zu lösen,
- damit homogene Polymermassen entstehen,
- die Polymermassen bei Verwendung von z. B. Benzoylaceton oder Dipivaloylmethan als Ligand transparent und bei Verwendung von Säurerestionen, wie Sulfaten, Nitraten, Halogeniden, Phosphaten u. a. farbig sein können,
- keine Lösungsmittel zum Mildern der Reaktivität der Lewis-Basen verwendet werden müssen,
- somit kein Erfordernis zusätzlicher Verarbeitungsschritte zur Entfernung des Lösemittels gegeben ist,
- dadurch keine eigenschaftsmindernden Hohlräume im Polymer entstehen,
- damit verbunden keine erhöhte Wasseraufnahmefähigkeit des Polymers zu verzeichnen ist,
- bei den an sich giftigen, hier als Initiatoren wirkenden Imidazol-Verbindungen, keine toxische Wirkung zu verzeichnen ist,
- die Spaltung der Lewis-Base-Metallverbindung bei Temperaturen oberhalb der Raumtemperatur, vorzugsweise zwischen 50 und 140 °C erfolgt,
- auch derartige "Einkomponentensysteme", bestehend aus Monomeren und Metallkomplex, unterhalb der Polymerisationsinitiierungstemperatur beliebig lange gelagert und geformt werden können und erst durch Erreichen der Initiierungstemperatur gehärtet werden.

Die Anwendung der Metallkomplexe mit der polymerisierbaren Verbindung ist mit oder ohne Zusatz von weiteren Additiven (z. B. Härter) möglich, d. h. die Polymermischungen sind multivariabel.

Der Beginn der Polymerisation, d. h. die Initiierungstemperatur ist durch die Wahl der Liganden, die Wahl der Lewis-Basen, bzw. die Wahl der Säurerestionen bestimmbar. Es reagieren Komplexe mit Anionen bei tieferen Temperaturen als Komplexe mit Chelatliganden. Der Einsatz von substituierten Lewis-Basen, z. B. alkylierten Imidazole hat ebenfalls Einfluß auf die Initiierungstemperatur, sie ist dann niedriger als bei der Verwendung von Imidazol als Lewis-Base.

Durch die geeignete Auswahl der Komplexe nach Art der Liganden, Lewis-Basen und Metall läßt sich die Polymerisationsinitiierungstemperatur in einer großen Breite variieren.

Dabei verläuft überraschenderweise die Reaktion zwischen dem Initiator und der polymerisierbaren Verbindung bei deutlich tieferen Temperaturen, als dies aufgrund von Komplexzersetzungstemperaturen, die aus der Literatur bekannt sind, zu erwarten wäre.

Weiterhin wird bei der Polymerisation von Epoxidverbindungen mittels der erfindungsgemäß eingesetzten Metall-Komplex-Verbindungen neben optimalen Gelzeiten eine reduzierte Wasseraufnahmefähigkeit und Acetonaufnahme gegenüber der Verwendung reiner Lewis-Basen wie Imidazol erreicht.

Weitere Vorteile des erfindungsgemäßen Verfahrens bestehen darin, daß eine Polymerisation vorzugsweise zwischen 50 und 140 °C erfolgt, daß diese "Einkomponentensysteme" unterhalb der Polymerisationsinitiierungstemperatur nach beliebig langer Lagerzeit geformt werden können und erst durch Erreichen der Initiierungstemperatur gehärtet werden und daß bei den an sich giftigen, hier als Initiatoren wirkenden Imidazol-Verbindungen keine toxische Wirkung zu verzeichnen ist.

Mit dieser Lösung ist die Möglichkeit gegeben, kostengünstige, umweltfreundliche und nichttoxische, latente Epoxidharzmassen mit optimalen Gelzeiten auf Grundlage von Metall-Komplex-Verbindungen herzustellen.

Diese Mischungen enthalten 100 Gewichtsteile der Epoxidverbindung und 0,01 bis 50 Gewichtsteile, bevorzugt 1 bis 10 Gewichtsteile einer Metallkomplexverbindung der allgemeinen Formeln

$ML_xB_y$ , oder $M[SX]_x B_z$ ,

versetzt wird und Energie zugeführt, wobei

- M ein Metallion der II. und III. Hauptgruppe sowie der Nebengruppen des Periodensystems,
- L ein chelatbildender Ligand aus der Gruppe der Dioxime, $\alpha$- und $\beta$-Hydroxycarbonylverbindungen oder enolisierbaren 1,3 Diketone,
- SR ein Säurerest einer anorganischen Säure

5

- B eine Lewis-Base
- x 1 oder 2
- y eine natürliche Zahl im Bereich von 1 bis 5
- z eine natürliche Zahl im Bereich von 7 bis 8

sind, mit der Maßgabe, daß die Lewis-Base keine mehrwertige phenolische Verbindung ist.

Aufgrund ihrer Eigenschaften empfehlen sich diese Mischungen, gegebenenfalls nach Zugabe von an sich bekannten Füll- und Zusatzmitteln, zur Herstellung von Klebe- und Dichtungsmassen. Sie dienen als Bindemittel für Formmassen, insbesondere für große Formteile die spannungsfrei härten.

Trotz der Ionen enthaltenden Metallkomplexverbindungen haben die erfindungsgemäß polymerisierten Epoxidverbindungen hervorragende elektrisch isolierende Eigenschaften. Daher eignen sich die Mischungen zur Herstellung von elektrischen Leiterplatten und als Bindemittel für Vergußmassen, insbesondere für den Elektro- und Elektronikbereich.

Darüberhinaus finden sie Verwendung bei der Herstellung von Hochleistungsverbundwerkstoffen wo sie, bedingt durch niedrige Temperaturspitzen bei der Polymerisation für einen spannungsfreien Verbund sorgen.

Beispiele

Die Erfindung soll nachfolgend an verschiedenen Ausführungsbeispielen erläutert werden:

Zur Ausführung werden jeweils 1 mol der Epoxidverbindungen mit 0,05 mol der jeweiligen Metallkomplexverbindung bei Raumtemperatur gemischt. Die Mischung wird geteilt:

Mit 100 g der Mischung wird die Gelierzeit nach DIN 16945, Abs. 6.3 (Verfahren A) bestimmt.

Ein Teil der Mischung wird zu Probekörpern zur Bestimmung der Wasser- und Acetonaufnahme gegossen und polymerisiert.

Der Rest der Mischung wird bei Raumtemperatur gelagert. Nach mehr als 6 Monaten Lagerzeit ist keine Polymerisation zu beobachten.

Die Ergebnisse sind in der folgenden Tabelle aufgeführt.

Dabei bedeuten

I  Imidazol

MI  2-Methylimidazol

RT  Raumtemperatur

Tg  (in den Beispielen 6 bis 8) Glasübergangstemperatur, bestimmt nach DIN 16946.

In der Tabelle sind für die einzelnen Versuche

- die jeweils verwendeten Epoxidverbindungen
- die jeweils verwendeten Metallkomplexverbindungen mit den durch sie in Abhängigkeit von der Temperatur erzielten Gelzeiten
- die prozentuale Wasseraufnahme der polymerisierten Epoxidverbindungen nach zweistündiger Erhitzung bei 100 °C
- die prozentuale Acetonaufnahme nach vierstündiger Erhitzung bei Siedetemperatur aufgeführt.

Tabelle

| Beispiel | Epoxidverbindung | Metallkomplex-verbindung | Temperatur °C | Gelzeit min | Wasserauf-nahme % | Acetonauf-nahme % |
|---|---|---|---|---|---|---|
| 1 | Diglycidylether d. Bisphenol-A | Co(benzoyl-acetonato)$_2$I$_2$ | 80 100 120 | keine Gelierung 10-fest 8-fest | 0,5 | 1,2 |
| 2 | N.N.N'N'-Tetra-glycidyldiamino-diphenylmethan | Co(benzoyl-acetonato)$_2$I$_2$ | 80 100 120 | keine Gelierung 15-fest 4-fest | 0,2 | 0,3 |
| 3 | Diglycidylether d. Bisphenol-A | Co(benzoyl-acetonato)$_2$MI$_2$ + 0,3 mol Diaminodi-phenylmethan | 100 120 | 3 h 13-fest | 0,3 | 0,35 |
| 4 | Diglycldylether d. Bisphenol-A | I$_8$CoSO$_4$ | 50 60 70 | 20 11 2 | keine Messung | |
| 5 | Diglycidylether d. Bisphenol-A | I$_8$CoCl$_2$ | 70 80 90 | 45 28 1 | keine Messung | |

EP 0 518 908 B1

Tabelle (Fortsetzung)

| Beispiel | Epoxidverbindung | Metallkomplex-verbindung | Temperatur °C | Gelzeit min | Wasserauf-nahme % | Aceton-aufnahme % | Tg °C |
|---|---|---|---|---|---|---|---|
| 6 | Diglycidylether d. Bisphenol-A | Co acetyl-acetonato$_2$I$_2$ | RT | keine Gelierung | 0,5 | 1,2 | 164 |
| | | | 80 | | | | |
| | | | 100 | 145-fest | | | |
| | | | 120 | 10-fest | | | |
| | | | | 6-fest | | | |
| 7 | Diglycidylether d. Bisphenol-A | Ni acetyl-acetonato$_2$I$_2$ | RT | keine Gelierung | 0,2 | 0,3 | 159 |
| | | | 80 | | | | |
| | | | 100 | 165-fest | | | |
| | | | 120 | 12-fest | | | |
| | | | | 6-fest | | | |
| 8 | Diglycidylether d. Bisphenol-A | Co acetato$_2$I$_2$ | RT | keine Gelierung | 0,3 | 0,35 | 153 |
| | | | 60 | | | | |
| | | | 80 | 90-fest | | | |
| | | | 90 | 20-fest | | | |
| | | | | 2-fest | | | |

**Patentansprüche**

1. Verfahren zur Polymerisation von Epoxidverbindungen mittels einer Metallkomplexverbindung, **dadurch gekennzeichnet**, daß 100 Gew.-Teile der Epoxidverbindung mit 0,01 bis 50 Gew.-Teilen einer Metallkomplexverbindung der allgemeinen Formeln

$ML_x B_y$ oder $M [SR]_x B_z$,

versetzt werden, und Energie zugeführt wird, wobei
- M ein Metallion der II. und III. Hauptgruppe sowie der Nebengruppen des Periodensystems,
- L ein chelatbildender Ligand aus der Gruppe der Dioxime, alpha- und $\beta$-Hydroxycarbonylverbin-dungen oder enolisierbaren 1,3 Diketone,
- SR ein Säurerest einer anorganischen Säure
- B eine Lewis-Base
- x 1 oder 2
- y eine natürliche Zahl im Bereich von 1 bis 5
- z eine natürliche Zahl im Bereich von 7 bis 8
sind, mit der Maßgabe, daß die Lewis-Base keine mehrwertige phenolische Verbindung ist.

2. Verfahren nach Anspruch 1, **dadurch gekennzeichnet**, daß eine Metallkomplexverbindung verwendet wird, die als Ligand Benzoylaceton, Acetylaceton oder Dipivaloylmethan enthält.

3. Verfahren nach einem der Ansprüche 1 bis 2, **dadurch gekennzeichnet**, daß eine Metallkomplexverbindung verwendet wird, die als Metallionen Cobalt-, Nickel-, Eisen-, Zink- oder Mangan-ionen enthält.

4. Verfahren nach einem der Ansprüche 1 bis 3, **dadurch gekennzeichnet**, daß eine Metallkomplexverbindung verwendet wird, die als Lewis-Base ein Pyridin, Imidazol, Tetrahydrofuran, Alkohol, Keton, Thioether oder ein Mercaptan enthält.

5. Verfahren nach einem der Ansprüche 1 bis 3, **dadurch gekennzeichnet**, daß eine Metallkomplexverbindung verwendet wird, die als Lewis-Base ein CH-acides Pyridin, Malonsäurediester oder -dinitrile, Acetessigsäureester, Cyanessigsäureester oder Nitromethan enthält.

6. Mischungen von Epoxidverbindungen und Komplexen, **dadurch gekennzeichnet**, daß sie 100 Gew.-Teile einer oder mehrerer Epoxidverbindungen und 0,01 bis 50 Gew.-Teile einer Metallkomplexverbin-dung der allgemeinen Formeln

$ML_x B_y$ oder $M [SR]_x B_z$

erhalten, wobei
- M ein Metallion der II. und III. Hauptgruppe sowie der Nebengruppen des Periodensystems,
- L ein chelatbildender Ligand aus der Gruppe der Dioxime, $\alpha$- und $\beta$-Hydroxycarbonylverbindun-gen oder enolisierbaren 1,3 Diketone,
- SR ein Säurerest einer anorganischen Säure
- B eine Lewis-Base
- x 1 oder 2
- y eine natürliche Zahl im Bereich von 1 bis 5
- z eine natürliche Zahl im Bereich von 7 bis 8
sind, mit der Maßgabe, daß die Lewis-Base keine merhwertige phenolische Verbindung ist.

7. Mischungen nach Anspruch 6, **dadurch gekennzeichnet**, daß sie auf 100 Gew.-Teile Epoxidverbindungen 1 bis 10 Gew.-Teile der Metallkomplexverbindung enthalten.

8. Verwendung der Mischungen nach einem der Ansprüche 6 und 7 zur Herstellung von Klebe- und Dichtungsmassen.

9. Verwendung der Mischungen nach einem der Ansprüche 6 und 7 als Bindemittel in Formmassen.

10. Verwendung der Mischungen nach einem der Ansprüche 6 und 7 als Bindemittel für Vergußmassen im Elektro- oder Elektronikbereich.

**11.** Verwendung der Mischungen nach einem der Ansprüche 6 und 7 zur Herstellung von elektrischen Leiterplatten.

**12.** Verwendung der Mischungen nach einem der Ansprüche 6 und 7 zur Herstellung von Hochleistungsverbundwerkstoffen.

**Claims**

**1.** A method for polymerizing epoxide compounds using a metal complex compound, **characterised in that** 100 parts by weight of the epoxide compound is mixed with from 0.01 to 50 parts by weight of a metal complex compound of the general formula

$ML_xB_y$ or $M [SR]_x B_z$,

and energy is provided,
- M being a metal ion of Group II or III or of the subgroups of the Periodic Table.
- L being a chelating ligand from the group of dioximes, alpha- and $\beta$-hydroxycarbonyl compounds or enolizable 1,3 diketones,
- SR being an acid residue of an inorganic acid
- B being a Lewis base
- x being 1 or 2
- y being a natural number ranging from 1 to 5
- z being a natural number ranging from 7 to 8
provided that the Lewis base is not a polyvalent phenolic compound.

**2.** A method according to Claim 1, **characterised in that** a metal complex compound is used which contains benzoyl acetone, acetylacetone or dipivaloylmethane as a ligand.

**3.** A method according to one of Claims 1 to 2, **characterised in that** a metal complex compound is used which contains cobalt, nickel, iron, zinc or manganese ions as metal ions.

**4.** A method according to any one of Claims 1 to 3, **characterised in that** a metal complex compound is used which contains a pyridine, imidazole, tetrahydrofuran, alcohol, ketone, thioether or a mercaptan as the Lewis base.

**5.** A method according to any one of Claims 1 to 3, **characterised in that** a metal complex compound is used which contains a CH-acidic pyridine, malonic acid diesters or dinitriles, acetoacetic esters, cyanoacetic esters or nitromethane as a Lewis base.

**6.** Mixtures of epoxide compounds and complexes, **characterised in that** they acquire [sic] 100 parts by weight of one or more epoxide compounds and 0.01 to 50 parts by weight of a metal complex compound of the general formula

$ML_xB_y$ or $M [SR]_x B_z$,

- M being a metal ion of Group II or III or of the subgroups of the Periodic Table.
- L being a chelating ligand from the group of dioximes, alpha- and $\beta$-hydroxycarbonyl compounds or enolizable 1,3 diketones,
- SR being an acid residue of an inorganic acid
- B being a Lewis base
- x being 1 or 2
- y being a natural number ranging from 1 to 5
- z being a natural number ranging from 7 to 8
provided that the Lewis base is not a polyvalent phenolic compound.

**7.** Mixtures according to Claim 6, **characterised in that** they contain 1 to 10 parts by weight of the metal complex compound per 100 parts by weight of epoxide compounds.

10

EP 0 518 908 B1

8. Use of the mixtures according to one of Claims 6 and 7 for the manufacture of adhesive and sealing materials.

9. Use of the mixtures according to any one of Claims 6 and 7 as a binding agent in moulding materials.

10. Use of the mixtures according to one of Claims 6 and 7 as a binding agent for casting materials in the electric or electronic field.

11. Use of the mixtures according to one of Claims 6 and 7 for the manufacture of electric printed circuit boards.

12. Use of the mixtures according to one of Claims 6 and 7 for the manufacture of high performance composite materials.

**Revendications**

1. Procédé de polymérisation de composés époxydes à l'aide d'un composé de complexe métallique, caractérisé en ce que 100 parties en poids du composé époxyde sont additionnées de 0,01 à 50 parties en poids d'un composé de complexe métallique de formule générale

$ML_xB_y$ ou $M[SR]_x B_z$,

et que l'on fournit de l'énergie, auquel cas
- M est un ion métallique des II et IIIèmes groupes principaux ainsi que des sous-groupes de la classification périodique des éléments,
- L est un ligand chélatant du groupes des dioximes, composés $\alpha$- et $\beta$-hydroxycarbonyles ou 1,3-dicétones énolisables,
- SR est un reste acide d'un acide inorganique,
- B est une base de Lewis,
- x est 1 ou 2,
- y est un nombre entier dans la gamme de 1 à 5,
- z est un nombre entier dans la gamme de 7 à 8, à condition que la base de Lewis ne soit pas un composé polyphénolique.

2. Procédé selon la revendication 1, caractérisé en ce qu'on utilise un composé de complexe métallique qui contient de la benzoylacétone, de l'acétylacétone ou du dipivaloylméthane en tant que ligand.

3. Procédé selon l'une des revendications 1 à 2, caractérisé en ce qu'on utilise un composé de complexe métallique qui contient des ions cobalt, nickel, fer, zinc ou maganèse en tant qu'ions métalliques.

4. Procédé selon l'une des revendications 1 à 3, caractérisé en ce qu'on utilise un composé de complexe métallique qui contient une pyridine, un imidazole, du tétrahydrofuranne, un alcool, une cétone, un thioéther ou un mercaptan en tant que base de Lewis.

5. Procédé selon l'une des revendications 1 à 3, caractérisé en ce qu'on utilise un composé de complexe métallique qui contient une pyridine à CH acide, des diesters ou des dinitriles maloniques, des esters acétylacétiques, des esters cyanoacétiques ou du nitrométhane en tant que base de Lewis.

6. Mélanges de composés époxydes et de complexes, caractérisés en ce qu'ils contiennent 100 parties en poids d'un ou de plusieurs composés époxydes et 0,01 à 50 parties en poids d'un composé de complexe métallique de formule générale

$ML_xB_y$ ou $M[SR]_x B_z$,

et que l'on fournit de l'énergie, auquel cas
- M est un ion métallique des II et IIIèmes groupes principaux ainsi que des sous-groupes de la classification périodique des éléments,

11

- L est un ligand chélatant du groupes des dioximes, composés $\alpha$- et $\beta$-hydroxycarbonyles ou 1,3-dicétones énolisables,
- SR est un reste acide d'un acide inorganique,
- B est une base de Lewis,
- x est 1 ou 2,
- y est un nombre entier dans la gamme de 1 à 5,
- z est un nombre entier dans la gamme de 7 à 8, à condition que la base de Lewis ne soit pas un composé polyphénolique.

7. Mélanges selon la revendication 6, caractérisés en ce qu'ils contiennent 1 à 10 parties en poids du composé de complexe métallique pour 100 parties en poids de composés époxydes.

8. Utiisation des mélanges selon l'une des revendications 6 et 7 pour la préparation d'adhésifs et de produits d'étanchéité.

9. Utilisation des mélanges selon l'une des revendications 6 et 7 comme liants dans des matières à mouler.

10. Utilisation des mélanges selon l'une des revendications 6 et 7 comme liants pour des masses d'encapsulation de haute qualité dans le domaine électrique ou électronique.

11. Utilisation des mélanges selon l'une des revendications 6 et 7 pour la confection de cartes imprimées électriques.

12. Utilisation des mélanges selon l'une des revendications 6 et 7 pour la préparation de matériaux composites à haute performance.